# EUROPEAN PATENT APPLICATION

(11) **EP 2 168 938 A1**
(43) Date of publication of application: **31.03.2010**
(21) Application number: 08016762.0
(22) Date of filing: 24.09.2008
(51) Int. Cl.: C07C 29/78, C07C 29/80, C07C 29/86, C07C 31/04

(54) **Process and plant to the production of methanol with improved distillation section**

(71) Applicant: METHANOL CASALE S.A., 6900 Lugano-Besso (CH)
(72) Inventor: Panza, Sergio, 6900 Lugano (CH); Capetti, Giovanni Luigi, 6900 Lugano (CH)
(74) Representative: Zardi, Marco

(57) **Abstract**

A process and a related plant for the synthesis of methanol, wherein a flash gas stream (103) obtained in the distillation section is treated to recycle the methanol contained therein and increase the production of refined methanol. Preferably the flash gas stream is washed with water in a counter-current column (C), recovering an acqueous solution of methanol (110) which is recycled back to the topping column (T) of the distillation section.

## Description

### Field of the invention

The present invention relates to a process and plant for the synthesis of methanol. More in detail, the invention discloses improvements to the distillation section of a methanol plant.

### Prior Art

Methanol is produced by catalytic reaction of a syngas containing hydrogen and carbon monoxide, at a high temperature and pressure, usually 200 to 300 °C and 40 to 150 bar. The product of the reaction is a stream of so-called crude methanol, which is subject to a distillation process to obtain refined pure methanol and separate water and by-products.

Generally speaking, the distillation section produces refined methanol and separates a flash gas, water, fuse oil and/or other by-products. Usually, the crude methanol is fed to a topping section, e.g. a topping column, to separate gas and light components, obtaining stabilised crude methanol; this stabilised crude methanol is then fed to a refining section to produce high-purity methanol.

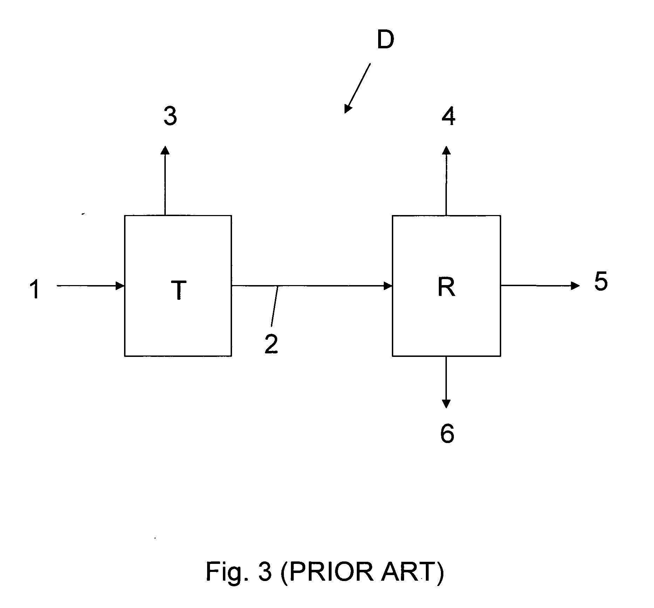

Fig. 3 shows a typical prior-art arrangement, where crude methanol 1 is sent to a topping section T, obtaining a stabilised crude methanol 2 and a flash gas 3; said methanol 2 is further sent to a refining section R obtaining refined methanol 4 and separating fusel oil 5 and bottom water 6. The topping section and the refining section form the distillation section D.

The flash gas separated in the topping section (stream 3) is available at a low pressure, typically less than 1 bar, and is used as fuel gas in the front-end of the methanol plant or, in some cases, is burnt in a torch. Burning the flash gas at such a low pressure, however, requires the use of specially adapted low-pressure burners.

There is a continuous effort aiming to increase the production of methanol plants, i.e. the rate of refined methanol obtained in the refining section, which is the valuable product of the process. This applies to new plants as well as to revamping or boosting existing plants.

As regards the boosting of existing or old installations, it is desirable to avoid relevant modification of the front end of the plant, for economic reasons. In some cases the front-end is already operated at its full capacity and cannot be boosted any longer.

### Summary of the invention

The problem underlying the invention is to find an effective way of increasing the quantity of refined methanol produced in a methanol plant.

The basic idea underlying the invention is to recover the methanol contained in the flash gas stream. Hence, the invention provides a process for the synthesis of methanol wherein crude methanol is produced in a synthesis section, and refined in a distillation section obtaining refined methanol, a flash gas stream and by-products, the process being **characterized in that** at least a portion of said flash gas stream is treated to separate a stream containing methanol from the flash gas, and said methanol-containing stream is recycled to the distillation section to increase the production of refined methanol.

According to one aspect of the invention, the treated flash gas stream is subject to a washing process, possibly with water recovered in the distillation section itself; an acqueous solution of methanol is obtained in said washing process, and said solution is recycled to the distillation section.

Preferably, the flash gas is washed in a counter-current arrangement and in at least one washing column, recovering an acqueous solution of methanol at the bottom of the column(s), and a combustible fuel gas at the top of the same. Said combustible gas contains mainly CO₂, H₂, CO and CH₄ and can be used as a fuel or sent to a torch.

According to another aspect of the invention, the pressure of the flash gas is raised before it is treated to recover methanol, that is to separate the aforesaid methanol-containing stream. Preferably the pressure of the flash gas is raised in an ejector. The motive stream of said ejector, in different embodiments, is any stream at a pressure greater than pressure of the flash gas; preferably the motive stream is a purge gas or tail gas from the synthesis section, or steam at a low pressure (2-20 bar) or mid-pressure (20-60 bar).

Usually, the distillation section comprises a topping section fed with the crude methanol and a refining section fed with stabilised crude methanol obtained in the topping section, the flash gas stream being obtained in said topping section. According to the invention, the flash gas stream, or at least portion thereof, is treated as described above, obtaining a methanol-containing stream which is recycled upstream the topping section, by mixing with the crude methanol inlet flow, or by direct feeding to the topping section itself.

As an example, in a preferred embodiment of the invention, the flash gas collected from top of a topping column is washed in a counter-current washing column; fuel gas is recovered at top of said washing column, while an acqueous solution of methanol is recovered at the bottom of the same column and recycled to the topping column. In accordance with the aforesaid aspects of the invention, the pressure of the flash gas stream may be raised with an ejector before it enters the washing column, and a condenser may also be provided between the ejector and the washing column.

A further object of the invention is a methanol plant adapted to operate according to the above process, comprising at least a synthesis section wherein crude methanol is produced, and a distillation section wherein said crude methanol is treated to refined methanol, obtaining also a flash gas stream and by-products, the plant being **characterized in that** it comprises recycle means disposed to collect at least a portion of the flash-gas stream, and adapted to separate a methanol-containing stream from the collected flash gas stream, and to recycle said methanol-containing stream to the distillation section, in order to increase the production rate of refined methanol.

In a preferred embodiment, said means are realized as a washing column, preferably fed with water, and more preferably operating in a counter-current arrangement.

In order to carry out the preferred embodiments of the inventive process, there is also provided an ejector disposed to raise the pressure of the flash gas stream, for example feeding the flash gas to said washing column. The motive stream of said ejector can be any suitable stream, such as purge gas, tail gas or steam. If steam is used, there is preferably a condenser downstream the ejector and upstream the washing column.

Still another object of the invention is a method for revamping a methanol plant, the plant comprising at least a synthesis section wherein crude methanol is produced, and a distillation section wherein said crude methanol is treated to refined methanol, obtaining also a flash gas stream and by-products, the method being characterized by the provision of means adapted to collect at least a portion of the flash-gas stream, to separate a methanol-containing stream from said flash gas stream, and to recycle said methanol-containing stream is to the distillation section, in order to increase the production of refined methanol. The related flow lines are also provided, according to the needs.

The main advantage is that the production rate of refined methanol is increased with the provision of relatively simple and low-cost equipments, without the need to modify the so-called front-end, namely the syngas compression section and the synthesis section. This is an important advantage because modification of the front-end is usually expensive.

The above advantage is particularly felt in cases of a revamping of a methanol plant having a front-end which is already operated at its full capacity and cannot be boosted any longer. In this case, the invention provides a cost-effective and easy way to increase the output of refined methanol.

Usually, the output of refined methanol increases by 1-2%; a less or more advantage, however, can be obtained depending on circumstances. In some cases, an advantage around 3% can be achieved. Generally, the invention is preferably applied to medium to large-sized plants.

Another advantage is that less pollutants and CO₂ are released to environment, because the methanol contained in the flash gas is recycled instead of burnt in a torch.

When the pressure of the flash gas stream is raised before separation of the methanol-containing stream, there is the further advantage that the fuel gas is made available at a pressure greater than that of the flash gas in the prior art (< 1 bar); this means that no special low-pressure burner is required.

These and other advantages of the invention will be more evident with the help of the following description of preferred and non-limiting embodiments.

### Brief description of the drawings

Fig. 1 shows, in a simplified manner, a diagram of the process in accordance with a first embodiment of the invention.
Fig. 2 shows a second embodiment of the invention.

### Detailed description of preferred embodiments

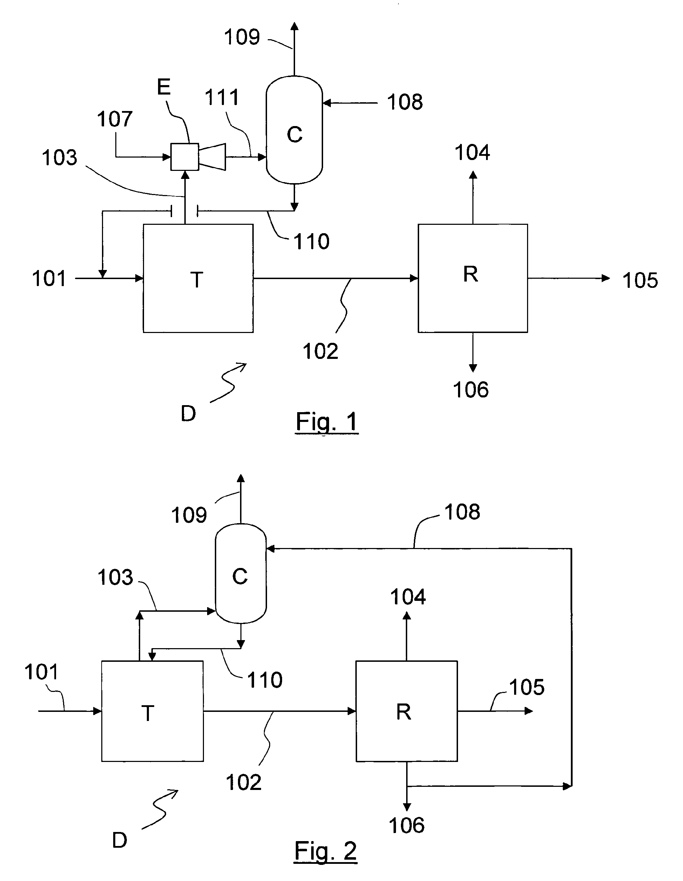

With reference to Fig. 1, a crude methanol 101 is obtained in a conventional methanol synthesis section (not shown), and sent to a topping section T, obtaining a stabilised crude methanol 102 and a flash gas stream 103. The stabilised crude methanol 102 obtained in the topping section T is further sent to a refining section R, obtaining refined methanol 104 and separating fusel oil 105 and bottom water 106. The topping section T and the refining section R are part of the distillation section D of a methanol plant.

The flash gas stream 103, or at least a portion thereof, is fed to a washing column C, via an ejector E powered by a motive stream 107. The washing column C yields a methanol-containing stream, in the example an acqueous solution of methanol 110, which is recycled to the inlet of the topping section T, by mixing with the crude stream 101. A fuel gas 109 is also recovered at top of the column C.

More in detail, the washing column C is preferably a counter-current washing column (absorber) fed with water 108, possibly the same water 106 (or a part thereof) recovered from the refining section. The solution 110 is recovered at the bottom of the column C, and recycled to topping section T via a pump or, as the case may be, thanks to the static head.

The motive stream 107 of the ejector E is any suitable, for example steam at a low pressure, such as 2 to 20 bar, or medium pressure such as 20 to 60 bar.

The advantage of using steam is that the compressed stream at the output of the ejector E (line 111), and then the fuel gas 109 recovered on top of the column C, is not diluted by the motive stream.

When the flow 107 is low or medium pressure steam, the output 111 of the ejector E is preferably fed to a condenser (not shown), before entering the column C, to condense the steam and remove absorbed methanol.

Thanks to the recycle of the methanol contained in the stream 110, the production rate of refined methanol (flow 104) is increased by some percent, usually around 1-2% or even more. A small increase of the reboiling duty to the topping section T may be required.

It should also be noted that the fuel gas 109 is available at a pressure greater than that of stream 103, due to pressure gain across the ejector E. The fuel gas 109, hence, does not require a special low-pressure burner. A conventional burner simpler and less expensive than in the prior art can be used.

In the simplified embodiment of Fig. 2, the stream 103 is sent directly to the washing column C, without raising its pressure. In the shown embodiment, a part of the bottom water 106, recovered in the refining section R, forms the water feed 108 of the washing column C, and the methanol-containing stream 110 formed in the column C is recycled directly into the topping section T.

It should be noted that recycling the flow 110 upstream the topping section T (Fig. 2) or directly to said topping section T (Fig. 3) are different embodiments of the inventive process, equally applicable to cases where the pressure of the flash gas is raised or not, before the methanol-recovering treatment in column C.

The topping section T and the refining section R are realized for example with one or more topping/refining column(s), according to the needs. It should be noted that the drawings are simplified and they do not show auxiliary equipments such as valves, etc... that are well known to a skilled person.

The invention relates also to a revamping method for a prior-art methanol plant. As a first example, a plant operating according to Fig. 3 can be modified to the new layout of Fig. 1 providing at least the washing column C, the ejector E raising the pressure of the flash gas, and the flow line 110 recycling the solution from bottom of the column C to the inlet of topping section T. The same plant can be modified to the layout of Fig. 2, providing at least the column C and the line 110 recycling the methanol solution from column C to the topping section T.

The following examples are given.

### EXAMPLE 1

The following table 1 is referred to a prior-art process as shown in Fig. 3. Table 2 is referred to the same process modified with the new layout of Fig. 1, wherein the flash gas 103 is compressed in an ejector E using tail gas from a membrane hydrogen-recovery unit as motive stream 107, and the compressed gas 111 is washed with water in the column C, recovering a methanol-containing stream 110 which is recycled with the crude methanol input 101.

The molar flow of refined methanol stream 104 is 7903.9 compared to 7809 of the prior-art stream 4, the input of crude methanol being the same. Increase of production rate is then 1.2%.

### EXAMPLE 2

A prior-art plant as in Fig. 3 is modified in accordance with the layout of Fig. 2, washing flash gas 103 with bottom water 108 recovered from a refining column. The produced refined methanol passes from 7809 to 7895.9 kg/s, increasing by 1.1%.

**Table 1**

| **Stream** | 1 | 2 | 3 | 4 | 5 | 6 |
|---|---|---|---|---|---|---|
| Vapour Fraction (mol) | 0.00 | 0.00 | 1.00 | 0.00 | 0.00 | 0.00 |
| Temperature (°C) | 49.5 | 86.3 | 45.0 | 45.0 | 90.4 | 118.8 |
| Pressure (bar abs) | 6.21 | 1.91 | 1.26 | 1.52 | 1.84 | 1.91 |
| Mass Flow (kg/h) | 320504.3 | 309949.8 | 10553.2 | 250214.7 | 1014.9 | 58676.9 |
| Molar Flow (kmol/h) | 11368.3 | 11102.0 | 266.2 | 7809.0 | 35.0 | 3256.4 |
| **Composition (% mol wet)** | | | | | | |
| CO | - | - | 0.020% | - | - | - |
| CO2 | 1.458% | - | 62.241% | - | - | - |
| H2 | 0.008% | - | 0.357% | - | - | - |
| H2O | 28.771% | 29.460% | 0.021% | - | 41.283% | 99.975% |
| CH4 | 0.003% | - | 0.118% | - | - | - |
| Ar | 0.001% | - | 0.052% | - | - | - |
| N2 | 0.006% | - | 0.239% | - | - | - |
| CH30H | 69.676% | 70.492% | 35.657% | 99.999% | 44.008% | 0.022% |
| Acetone | 0.001% | - | 0.022% | - | - | - |
| Dim-Ether | 0.013% | - | 0.548% | - | - | - |
| Ethanol | 0.032% | 0.032% | - | 0.001% | 9.955% | 0.003% |
| 1-propanol | 0.015% | 0.015% | - | - | 4.754% | - |
| M-Formate | 0.017% | - | 0.724% | - | - | - |

**Table 2**

| **Stream** | 101 | 102 | 103 | 104 | 105 | 106 |
|---|---|---|---|---|---|---|
| Vapour Fraction (mole) | 0.00 | 0.00 | 1.00 | 0.00 | 0.00 | 0.00 |
| Temperature (°C) | 49.5 | 86.8 | 45.0 | 45.0 | 91.2 | 118.8 |
| Pressure (bar abs) | 6.21 | 1.91 | 1.26 | 1.52 | 1.84 | 1.91 |
| Molar Flow (kmol/h) | 320504.3 | 321621.7 | 10617.6 | 253255.4 | 996.9 | 67424.0 |
| Mass Flow (kg/h) | 11368.3 | 11679.3 | 267.6 | 7903.9 | 35.0 | 3742.6 |
| **Composition (%mol wet)** | | | | | | |
| CO | - | - | 0.027% | - | - | - |
| CO2 | 1.458% | - | 62.008% | - | - | - |
| H2 | 0.008% | - | 0.358% | - | - | - |
| H2O | 28.771% | 32.170% | 0.023% | - | 44.996% | 99.999% |
| CH4 | 0.003% | - | 0.118% | - | - | - |
| Ar | 0.001% | - | 0.052% | - | - | - |
| N2 | 0.006% | - | 0.238% | - | - | - |
| CH30H | 69.676% | 67.785% | 35.632% | 99.999% | 40.093% | 0.001 % |
| Acetone | 0.001% | - | 0.029% | - | - | - |
| Dim-Ether | 0.013% | - | 0.581% | - | - | - |
| Ethanol | 0.032% | 0.031% | - | 0.001% | 10.140% | - |
| 1-propanol | 0.015% | 0.014% | - | - | 4.771% | - |
| M-Formate | 0.017% | - | 0.932% | - | - | - |

## Claims

1. A process for the synthesis of methanol, wherein crude methanol (101) is produced in a synthesis section, and refined in a distillation section (D) obtaining refined methanol (104), a flash gas stream (103) and by-products (105, 106), the process being **characterized in that** at least a portion of said flash gas stream (103) is treated to separate a methanol-containing stream (110) from the flash gas, and said methanol-containing stream (110) is recycled to the distillation section (D) to increase the production of refined methanol.

2. A process according to claim 1, wherein said at least a portion of flash gas (103) is treated with a washing process, obtaining an acqueous solution of methanol (110), and said solution is recycled to the distillation section.

3. A process according to claim 2, wherein the washing of flash gas stream (103) is carried out with water (108) recovered from the crude methanol (101) in said distillation section (D).

4. A process according to claim 2 or 3, wherein the washing of flash gas stream (103) is made in counter-current and in at least one washing column (C), recovering said solution of methanol (110) at the bottom of the column(s), and obtaining a combustible fuel gas (109) at the top of said column(s).

5. A process according to any of claims 1 to 4, wherein the pressure of said at least a portion of flash gas stream (103) is raised before the flash gas is treated to separate said methanol-containing stream.

6. A process according to claim 5, wherein the pressure of the flash gas (103) is raised in an ejector (E).

7. A process according to claim 6, wherein the motive stream (107) of said ejector (E) is low-pressure or medium-pressure steam, and the output flow (111) of the ejector is subject to condensation before the treatment to recover said methanol-containing stream.

8. A process according to any of the preceding claims, wherein said flash gas stream (103) is obtained in a topping section (T) fed with the crude methanol (101) and producing said flash gas stream and a stabilised crude methanol (102) directed to further refining, and wherein the recovered methanol-containing stream (110) is mixed with the crude methanol (101) entering the topping section, or recycled directly to the topping section.

9. A plant for the synthesis of methanol adapted to operate according to the process of any of claims 1 to 8, the plant comprising at least a synthesis section wherein a syngas is converted into crude methanol (101), and a distillation section (D) wherein said crude methanol (101) is refined, obtaining refined methanol (104) and also a flash gas stream (103) and by-products, the plant being **characterized in that** it comprises recycle means (C, E) disposed to collect at least a portion of said flash-gas stream (103), and adapted to separate a methanol-containing stream (110) from the collected flash gas stream, and to recycle said methanol-containing stream to the distillation section (D).

10. A plant according to claim 9, wherein the distillation section (D) comprises a topping section (T) fed with the crude methanol (101), and a refining section (R), and said recycle means comprise a counter-current, water-fed washing column (C), receiving the flash gas stream (103) collected from the topping section (T), and wherein the plant further comprises a flow line for recycling an acqueous solution of methanol (110) from said washing column to said topping section.

11. A plant according to claim 10, **characterized in that** it further comprises an ejector (E) raising the pressure of the collected flash gas stream (103) upstream said washing column (C).

12. A method for revamping a methanol synthesis plant, the plant comprising at least a synthesis section wherein crude methanol (101) is produced, and a distillation section (D) wherein said crude methanol is treated to refined methanol (104), obtaining also a flash gas stream (103) and by-products, the method being **characterized by** the provision of recycle means (C, E) and flow lines adapted to collect at least a portion of said flash-gas stream (103), to separate a methanol-containing stream (110) from said at least a portion of flash gas stream, and to recycle said methanol-containing stream (110) to the distillation section (D).
